# EUROPEAN PATENT SPECIFICATION

(11) **EP 4 401 675 B1**
(45) Date of publication and mention of the grant of the patent: **21.01.2026**
(21) Application number: 21778363.8
(22) Date of filing: 13.09.2021
(51) Int. Cl.: A61F 2/07, A61F 2/97, A61F 2/04

(54) **SUCTION STENT**
SAUGSTENT
ENDOPROTHÈSE D'ASPIRATION

(43) Date of publication of application: 24.07.2024
(60) Divisional application: 25220945.7
(73) Proprietor: Vac Stent GmbH, 36043 Fulda (DE)
(72) Inventor: HEISS, Constantin, 72072 Tübingen (DE); WÄCHTERSBACH, Timo, 36088 Hünfeld (DE); ERBE, Konrad, 98617 Rhönblick (DE)
(74) Representative: Maiwald GmbH
(86) International application number: PCT/EP2021/075097
(87) International publication number: WO 2023/036445

(56) References cited:
- WO-A1-2004/110311
- WO-A1-2015/086037
- US-A- 5 290 295
- US-A- 5 628 788
- US-A1- 2019 183 666
- US-A1- 2020 060 807

## Description

### Technical field

The present invention relates to a stent for introduction into a hollow organ, in particular the gastrointestinal tract, of a human or animal patient. Such stent may particularly be used to provide a vacuum sealing of leaks to a particular anatomic region in the hollow organ, e.g. for the treatment of local anastomosis insufficiencies.

### Background of the invention

Leaks in surgical sutures (anastomoses) in the gastrointestinal tract are most dangerous and hence constitute one of the most significant complications after operations in the abdominal region. In the event of leaks, the contents of the stomach or intestine pass into the abdominal cavity and thus lead to peritonitis which even nowadays is still fatal in about 20% of cases. Treating such a leak is dependent on the exact location and the damage which has already occurred owing to the escaped contents of the intestine. In the best case, healing of the suture is delayed and the functional result of the operation, e.g. continence, is impaired. Frequently, however, considerably more invasive measures such as a reoperation with removal of the intestinal continuity and fitting of a colostomy are required in order not to endanger the patient's life. The process of fitting a colostomy can only be reversed in a fraction of cases.

Attempts to seal anastomosis insufficiencies, e.g. using an endoscopically placed endoluminal covered stent or other conventional stents, have been found to be frequently unsuccessful in adequately sealing the suture in hollow organs. This insufficiency may generally be attributed to the incongruency of the applied stent with the irregularly shaped intestinal wall. Self-expandable stents with high restoring forces cannot be used to achieve complete sealing in the region of leaky sutures either as this could lead to further damage or even bursting of the suture.

Furthermore, even if, in exceptional cases, complete sealing of the defect is actually achieved, the contents from the hollow organ that have entered the region of the suture, e.g. the contents of the intestine, cannot be drained away. Formation of an abscess at the suture therefore virtually inevitably occurs, in particular in the gastrointestinal tract, thereby leading to further aggravation of the local pathological condition and the medical status of the patient.

To improve the sealing towards the wall of the hollow organ, e.g. the intestine, the implementation of a porous foam material has been suggested, which may be arranged at an exterior of a stent body and may be held in place by means of radially outward pressure exerted by the stent body. Drainage of detrimental contents from the respective hollow organ may furthermore be achieved by providing a cannula at an exterior of the stent body, e.g. within the porous material. By applying a vacuum, this may furthermore result in an improved sealing of the suture or may even replace the necessity of applying a suture to close a lesion until said lesion has healed.

After prolonged usage of stents, however, it has been found that local tissue in-growth may occur, wherein, in particular, the porous material and the typically mesh-like stent body appear to be most susceptible. Due to the facilitation of the healing process of (leaking) sutures and hence the preferably temporary nature of the stent application, removal or retraction of the stent may constitute further challenges in avoiding damage to the surrounding tissue.

US 2020/060807 describes a stent for introduction into a hollow organ with a stent portion imbedded within a layer. Onto that layer another layer is attached.

Therefore, a need exists to further improve current stents in terms of patient safety and treatment efficacy both during and after application of the stent.

### Summary of the invention

Starting from the known prior art, it is therefore an object of the present invention to provide a stent which effectively seals any local defects, e.g. leaky surgical sutures, and at the same time facilitates removal after prolonged usage. Preferably, such stent also enables effectively removing any accumulations of fluid at such defects, in hollow organs of the human or animal body.

This object is achieved by the stent of the present invention according to the independent claims. Preferred embodiments are depicted in the dependent claims, the description and the Figures.

Accordingly, a stent for introduction into a hollow organ of the human or animal body, preferably into the gastrointestinal tract, in particular the intestine, is suggested. The stent comprises a radially expandable body having a wall defining an inner fluid passageway from one end of the body to a longitudinally opposing end of the body. Furthermore, the stent comprises a liquid-tight and flexible first layer, wherein the first layer covers a surface of said wall along its entire circumference and along a first predefined region in a longitudinal direction of said body, and a resilient porous second layer, wherein the second layer covers an outer surface of said wall along its entire circumference and along a second predefined region in a longitudinal direction of said body and at least partially covers the first layer. The second layer is mechanically secured to the body as defined by claim 1.

Optionally, the first layer may be arranged at said wall surface and extends at least partially through said wall in a radial direction

By means of the radial extension of the first layer through the wall any holes or cavities present in the wall may be filled, such that the wall is at least partially embedded in the first layer. Thereby, a direct contact with the wall is ensured and maintained during deployment of the stent, such that the sealing function of the first layer with respect to the fluid passageway and towards the exterior is improved. Furthermore, the radial extension through the wall provides mechanical stability of the first layer by not only partially increasing the thickness of the first layer, but also by securing the first layer to the wall in a radial, longitudinal and circumferential direction.

Moreover, by protruding through or penetrating the wall, in-growth of local tissue is impaired. For example, the stent body may be mesh-shaped, i.e. have a wire structure, such that the radial extension of the first layer fills said mesh-shape. Thereby, local tissue is essentially unable to grow through or interlace with the mesh-shape structure of the stent body. This equally applies to other types of stent bodies having e.g. a porous structure or (continuous) hole pattern. Accordingly, removal of the stent is facilitated and potential tissue damage may be reduced upon removal while the sealing function during application of the stent is ensured due to the improved mechanical or structural stability of the first layer.

The mechanical securing of the second layer to the body as defined by claim 1 ensures that even if limited local tissue in-growth occurs through the porous structure, the stent may be safely removed and the second layer does not reside in the patient upon retraction of the stent. During application the mechanical securing of the second layer ensures proper positioning of the second layer, such that a sealing towards the inner wall of the hollow organ at the site of a lesion or suture is improved and maintained even during movement or contractions of the surrounding tissue.

Hence, both the configuration of the first layer radially extending through the wall and the securing of the second layer to the stent body achieve an effective sealing of local tissue defects and facilitate the removal of the stent after prolonged usage. They may synergistically improve the safety of the stent during the application and the removal, such that the stent according to the invention may preferably comprise both the advantageous configuration of the first layer and the advantageous configuration of the second layer.

The fluid passageway of the body or stent body (referred to analogously throughout the present invention) may be understood as a through-channel or inner cavity with opposing openings. The body hence forms a hollow body having a lumen and being open in the longitudinal direction, wherein a fluid may enter the passageway via one end and may exit the passageway via the opposing end. The body is preferably of an essentially cylindrical or tubular shape, but may also comprise other cross-sectional shapes, such as an ellipsoid shape, at least for one or more regions of the body. While the body is preferably an elongated body with an essentially continuous longitudinal extension, one or more curvatures may be present, which may e.g. enable an adaptation of the stent to a particular anatomy corresponding to the respective application.

Preferably, the radially expandable body is of a mesh-shape, which facilitates expansion and compression of the stent and provides an extent of adaptability to the anatomic structure of the application region, e.g. the intestinal wall. The body may be formed of a shape memory alloy, e.g. nitinol, which further facilitates collapsing the stent for delivery to a target lesion or suture via a delivery system and catheter. The body may also be configured to be self-expanding, which may be facilitated by using such shape memory alloy or other metal. Such material furthermore provides resilience of the body, thereby providing an adaptability to the local anatomy at the application site and, depending on the dimensioning of the stent body; achieving that the stent is held in place by means of radially and/or longitudinally exerting forces. Although a shape memory alloy may be preferable for structural stability, the stent body may alternatively also be formed of a self-expandable plastics material.

The luminal diameter of the stent according to the invention, i.e. the radially expandable body is preferably in the range of about 10 to 50 mm, preferably 15 to 35 mm, in particular 15 to 30 mm, most particularly preferably it is about 28 mm (for example in applications in the colon region) or about 21 mm (for example for use in the esophagus). In any case, the diameter of the stent is selected such that, depending on the area of application, the passage of corresponding material through the respective hollow organ - the passage of food in the case of the intestinal tract - is not obstructed. While the above dimensions may apply to the expandable body as a whole, at least one of the opposing ends or end regions may have a larger radial extension or said dimensions may be met by the opposing ends or end portions and the portion between said opposing ends may be dimensioned smaller.

The liquid-tight and flexible first layer may also be resilient and/or compressible or collapsible, such that the integrity of the material of the first layer is not adversely affected in a collapsed state and the material preferably facilitates the expansion of the first layer in an essentially homogeneous manner, preferably also facilitating the expansion or at least not impairing the expansion of the body. The liquid-tight first layer ensures that liquid outside of the stent body may not enter the fluid passageway and vice versa, such that bodily fluids of the hollow organ may not inadvertently invade surrounding organs or enter the blood circulation.

Preferably, the first layer is fluid-tight or air-tight. Should the stent be equipped with a drainage means such as a cannula, which is arranged outside of the first layer, this provides that a vacuum may be applied between the first layer and the inner wall of the hollow organ. Any contents leaking into this intermediate space may hence be effectively drained by means of a negative or suction pressure and a (small) vacuum may furthermore facilitate sealing of a lesion or suture, thereby expediting the healing process.

The first layer may be formed of or comprises a plastics or polymer material, preferably being selected from the group comprising polyurethanes, latex and silicone. Preferably, the first layer is silicon-based.

The resilient porous second layer is preferably shapeable and/or compressible while in the absence of compressive forces returning to its original non-compressed state. The second layer may enclose the stent body and may e.g. be tubular-shaped having a central through-hole accommodating the stent body and the first layer. The second layer may be formed of a closed-pore material, i.e. in the form of a foam, or an open-pore material, i.e. in the manner of a sponge. Preferred materials for this purpose are plastics material foams, for example including or consisting of polyurethanes, polyvinyl alcohols or mixtures of such plastics materials.

The second layer preferably comprises a thickness of about 5 mm to about 20 mm, preferably of about 5 mm to about 10 mm, the exact dimensioning being dependent on the anatomic dimensioning at the application site and the physical requirements in view of e.g. the required resilience and the necessary bridging between the stent body and the inner wall of the hollow organ.

The entire construction of the stent according to the invention is preferably completely expandable and can be brought to the application site in the organ, in particular the gastrointestinal tract, preferably the esophagus, intestine, primarily rectum, sigma, colon descendens or colon transversum, by conventional application measures.

The first layer may be arranged at an inner wall surface or an outer wall surface and extends in a radial direction so as to fill at least the space between said surfaces in order to embed and mechanically secure the stent body with the first layer. Preferably, the first layer is at least arranged at the outer wall surface of the stent body. More preferably, the first layer covers an inner surface and an outer surface of said wall. Thereby, the stent body, i.e. the wall of the stent body is fully embedded by the first layer. As opposed to implementations with liquid-tight foils, such configuration provides a thicker material layer, effectively reducing leakage and inhomogeneities and ensuring that the first layer is fully secured to the stent body, which may preferably be of a mesh shape. Furthermore, such embedded configuration and corresponding structural linking may avoid folding of the first layer, providing a more homogeneous outer profile and reducing the potential occurrence of wear during application.

Preferably, the first predefined region and/or the second predefined region corresponds to a region of the body having an essentially continuous cross-sectional area. Such essentially continuous cross-sectional area may be provided for the entire region between the opposing ends and may e.g. be of a tubular, circular, or ellipsoid shape. However, intermediate constrictions may be present, e.g. to ensure mechanical stability or to provide an adaptation to an anatomical structure. The cross-sectional area may relate to the wall, the passageway, or both. The matching of the respective predefined region to the continuous cross-sectional area facilitates manufacturing and may furthermore avoid bulging, folding, or wrinkle formation of the respective layer during expansion and/or application of the stent.

The first predefined region and the second predefined region may hence essentially correspond to each other. Alternatively, the first layer may also longitudinally extend beyond the second layer at a respective end or end region of the stent body. For example, the stent may comprise a drainage means, e.g. in the form of a cannula, which is accommodated alongside the stent body, yet which may be guided through the stent body at a respective end region. To ensure that the fluid passageway of the stent body is liquid-tight at this respective end region whilst the drainage means extends through the corresponding wall, the first layer may not be applied at said end region and other means for maintaining the liquid-tight configuration may be provided at said end region. As will be described in detail herein below, for example, a liquid-tight and preferably fluid-tight foil may be provided at said end region at an inner wall surface and being continuous with the first layer, wherein the drainage means or cannula is arranged outside of the foil and, at least in part, between the wall and the foil in said end region.

The first predefined region and/or the second predefined region may be longitudinally delimited by at least one end region of the body, wherein the at least one end region has an enlarged radial extension.

At least one of the end regions, preferably both opposing end regions may e.g. have a varying cross-sectional area or shape and/or exhibit a radial increase directly adjacent to a region of the stent body having an essentially continuous cross-sectional area. In the event that one of the end regions is configured to receive a drainage means or cannula, the first layer may be delimited by said end region and instead a foil, e.g. a tubular foil may be arranged at the inner wall surface, as described in the above. Such foil is preferably fluid-tight, i.e. air-tight and water-tight, and may be formed e.g. of a polyurethane, latex and/or silicone or silicon-based material. The foil may be fixed to the first layer by means of a sealant, such as a silicone, hydrocolloid or lyogel, in particular a hydrogel in order to form a homogeneous and structural stabile sealing structure.

The second layer may at least partially cover a respective end region as long as the overall radial extension of the second layer is essentially constant. For example, where the end region gradually increases its radial extension, the thickness of the second layer may be accordingly reduced until reaching zero. This provides a smoother transitioning between the (outer) second layer and the radially protruding end region.

The respective end region may have a mushroom shape, a dome shape, a toroidal shape, or a donut shape in a longitudinal section of the body. The overall body may accordingly have a barbell shape.

The particular shapes comprise a rounded surface having no sharp edges, thereby reducing the risk of neighboring tissue damage, e.g. upon deployment or dislodgement of the stent body. Furthermore, the rounded shapes provide an improved fitting to the local anatomy of the application site, i.e. better adapt to the inner wall of the hollow organ while exhibiting a resilience securely holding the stent in place at the application site.

In this regard, an enlarged radial extension at both opposing end regions also enables that the stent may be placed in such a manner that the lesion or suture is positioned between the respective end regions and the end regions thereby facilitate or improve a targeted application of a vacuum or drainage at this particular location.

Both the first predefined region and the second predefined region may furthermore be delimited by both of the opposing end regions. The first predefined region and the second predefined region may hence comprise essentially the same longitudinal extension. However, as described above, the second layer may at least partially cover a respective end region as long as its thickness is accordingly reduced. Furthermore, a respective end region may be configured to receive and accommodate a drainage means, wherein instead of the first layer a liquid-tight foil may be implemented within the fluid passageway or below or at the inner wall surface. However, the opposing end region not receiving such drainage means may optionally be covered and embedded, at least in part, with the first layer.

The delimitation of the second layer by both of the opposing end regions may furthermore provide that the second layer is mechanically secured in a form fitting manner by the opposing end regions in the longitudinal direction. Depending on the dimensioning and type of material for the second layer, a loose form fitting may be provided, such that e.g. radial or longitudinal forces exerting upon the second layer may still result in a corresponding displacement. The delimitation, however, provides that, at least during deployment of the stent, the second layer is biased between the opposing end regions so as to ensure proper application of the stent to the target site.

Depending on the dimensioning and the material used for the second layer, the second layer may also to some extent be secured to the stent body (or first layer) by means of a friction fit or press fit, e.g. in a rotational direction and/or longitudinal direction.

According to the invention, in order to provide an improved fixation of the second layer to the stent body, the second layer is mechanically attached to the body by means of at least one thread, a first thread portion being arranged in the second layer so as to provide at least one eyelet at an outer surface of said second layer and a second thread portion connecting the at least one eyelet to a respective connection point at an adjacent end region of the body not being covered by the second layer.

The adjacent end region is preferably also not covered by the first layer, e.g. when receiving and accommodating a drainage means. Thereby, connecting the second thread portion to the respective end region, which is preferably a proximal and/or nearest end region, may be facilitated. To improve structural stability of the second layer, the at least one eyelet is preferably space apart from an end face of the second layer corresponding to the respective end region in a longitudinal direction. Preferably, the at least one eyelet may be arranged between about 5 mm to about 20 mm from the respective end face, preferably between 8 mm and 15 mm or about 10 mm.

The thread may be formed of a biocompatible yet non-biodegradable material, e.g. a suture material. Preferably, the thread is formed of polyethylene (PE), polypropylene (PP) or polytetrafluoroethylene (PTFE). The connection with the connection point may be provided with a respective knot at the connection point, e.g. at intersecting struts of a mesh-shaped body, or via a loop so as to provide a laced fixation. By means of the connection, the second layer is secured both in a radial and longitudinal direction.

Preferably, at least two eyelets are provided by the first thread portion, wherein said eyelets are circumferentially spaced apart. In this manner, potential movement of a portion of the second layer, i.e. in the longitudinal and/or radial direction, may be reduced. The second thread portion may connect each eyelet individually to a respective connection point or may connect the eyelets with each other, e.g. via one or more connection points using a corresponding loop and lacing technique.

Preferably, the eyelets are equally spaced apart along the circumference and/or between three and six or four eyelets are provided by the first thread portion. Both the equal spacing and the plurality of eyelets provide that stresses acting on the second layer due to the mechanical attachment may be reduced and constrictions may be effectively avoided. For example, the provision of four eyelets at about 90° displacement along the circumferential direction enables that the second layer is pulled towards the stent body in an evenly distributed manner without the mechanical attachment becoming too laborious. However, further eyelets may be provided, depending on the requirements of the stent and the configuration of second layer being used.

A variety of connection methods may be provided, wherein loops and/or knots may be used for the plurality of connection points and eyelets. Preferably, the second thread portion alternates between connection points and eyelets that are adjacent to each other and/or each connection point is connected to two adjacent neighboring eyelets by means of the second thread portion.

Each eyelet may hence also be connected to two adjacent connection points. The alternating pattern is to be understood essentially as a zig-zag pattern, e.g. between a circumferential line formed by the connection points and a circumferential line formed by the eyelets. The respective connections may be formed by knots or by loops, e.g. by lacing the second thread portion through the eyelet and by guiding the second through portion e.g. around a connection point. If the connection point is formed by an intersection of connecting struts in a wired mesh-shape of the stent body, the second thread portion may hence be guided from a respective eyelet around an adjacent strut or intersection of struts at the end region and may be guided further towards a further adjacent eyelet, thereby forming a loop between said eyelets via the connection point. The connection point may also be specifically formed for such purpose at the outer surface of the stent body, e.g. in the form of a retaining element, a rounded protrusion having or defining a kerf or slit, or a corresponding eyelet.

As described above, the end region may also have a larger radial extension, e.g. may be mushroom shaped and extending radially beyond a tubular region of the stent body having an essentially continuous cross-sectional area. In such configuration, the connection may be facilitated, since this reduces the risk of constricting the second layer (and potentially the stent body and/or first layer) and this provides that the connection points may be more easily accessible. Preferably, although a radial extension may be advantageous, the connection point is chosen or arranged so as to minimize the radial extension of the second thread portion. This facilitates the deployment and functioning of the stent and reduces any potential adverse effects to the surrounding tissue structure in the implanted state.

To further improve the force distribution and provide a more even and homogeneous attachment of the second layer to the stent body, each connection point may be arranged essentially equidistantly to two adjacent neighboring eyelets. The connection points are hence preferably also equally spaced-apart along the circumference of the stent body and are arranged essentially in the middle between two adjacent neighboring eyelets, yet with a longitudinal offset. The equidistant arrangement of the connection points avoids a biasing of individual eyelets (and hence the second layer) towards a particular connection point.

Between adjacent eyelets the first thread portion may be arranged within the material of the second layer. The first thread portion may hence be guided through the second layer and may be embedded therein, such that only the eyelets formed by the first thread protrude out of the second layer. Such configuration has the advantage that larger first thread portions between the eyelets that are adapted to both a collapsed or compressed state and an expanded state of the stent are retained in the second layer and hence do not inadvertently form loops, bends or other folds that may impair proper expansion and functionality of the stent, e.g. by potentially interacting with the anatomic structure at the application site or with other components of the stent. Furthermore, the inner first thread portions reduce the amount of thread portion being in contact with the surrounding tissue, such that friction or incisions towards the tissue may be reduced. The potential of tissue invasion or in-growth around the first thread portion is furthermore also reduced.

While the first thread portion and the second thread portion may be formed as separate threads, the first thread portion and the second thread portion are preferably formed of a single thread. In other words, the first thread portion may be arranged at and/or in the second layer so as to provide the respective one or more eyelets and continue as the second thread portion, which connects said eyelet(s) to the respective connection point(s). For example, the first thread portion may be guided through the second layer, wherein a leading end only protrudes out of the second layer to form a respective eyelet, e.g. by forming an outer loop and wherein the lagging end forms a first eyelet by means of one or more ties with the first thread at the lagging end. After the last eyelet, the leading end of the first thread then continues as the second thread portion, connecting the respective eyelet(s) via the one or more connection points, e.g. in an alternating zig-zag pattern.

The use of one and the same thread, i.e. a single piece of thread, enables that fewer knots may be required, which may be physiologically advantageous, since the occurrence of a biasing of the second layer and/or the stent body may be reduced and fewer knots are present, such that a smoother outer surface of the stent is provided. Furthermore, the use of a single thread may be advantageous for the overall structural stability of the thread.

Although the attachment of the second layer may be provided at either end region of the stent body, the adjacent end region is preferably configured for accommodating a cannula. The adjacent end region may hence correspond to a proximal end region of the stent. This is particularly advantageous upon removal and retraction of the stent, since frictional and/or tensional forces particularly occur at this end and the second layer is hence directly attached to said end to reduce potential leverage or a longitudinal shifting or folding of the second layer towards the distal end.

Accordingly, the stent may further comprise a cannula being arranged between the second layer and the first layer and essentially being accommodated outside of the body. The cannula or a drainage means in general may be connected to the second layer and/or to the stent body to ensure proper positioning of the cannula within the stent, i.e. between the opposing ends or end regions. The cannula may e.g. be couplable to a vacuum or negative pressure source to provide a vacuum between the first layer and an inner wall of the hollow organ in the implanted and deployed state. However, other functions may optionally, preferably additionally, be provided using the cannula, e.g. by enabling a rinsing or flushing of the application site, e.g. with a saline or other biologically compatible fluid, or by applying a liquid or gel-like tissue sealant to facilitate healing of a lesion or leaky suture.

The cannula may be received via an end region having a larger radial extension than the fluid passageway, so as to minimize the radial dimensioning of the stent and the passageway. For example, the end region may be mushroom-shaped or dome-shaped, wherein the cannula is introduced via the passageway and extends through the wall at said end region so as to be received and accommodated along an outside of the stent body between the end regions.

According to a further aspect of the invention, a delivery system for delivering and deploying a stent to a target anatomic region is suggested, comprising a catheter having a stent according to invention in a compressed state. Preferably, the delivery system is configured, dimensioned and adapted to enable a navigation and delivery of the stent to an application site in the gastrointestinal tract of a patient, preferably the esophagus, intestine, primarily rectum, sigma, colon descendens or colon transversum.

Preferably, the catheter comprises a distal end cap made of a flexible material, wherein the end cap defines an inner cavity and has a convex rounded end surface in a longitudinal direction. The end surface has at least three, preferably four slits from its outer surface towards the inner cavity that are equally spaced apart in the circumferential direction.

Thereby, a small opening may be formed for a guide wire and a corresponding navigation or end bead may be reduced in diameter, which is advantageous for the patient and in view of preferred minimally-invasive methods. By means of the slits, a corresponding number of flaps are formed at the end surface, which, due to the flexible material, may be biased towards an open or radially and longitudinally outward position so as to provide a continuous through-hole for a compressed and/or collapsed stent. In this regard, a configuration with four slits provides even more flexibility and reduces forces counteracting the deployment and guiding of the stent out of the catheter.

Compared with e.g. a single slit configuration, which provides a limited opening of the end surface towards the inner cavity and hence requires a larger slit and end surface, the end cap having three or more slits hence may be dimensioned smaller. This also applies compared with pivotable configurations, wherein a closure at the end cap is required to be opened beyond the radial extension of the actual opening and hence requires a larger dimensioning.

Compared with such configurations, the end cap according to the invention also significantly facilitates the maneuvering of the delivery system, since the slits readily respond to an advancing of the stent out of the catheter and require no further actuation.

A respective shape of the end surface defined by two circumferentially adjacent slits may furthermore be truncated at its free end. In other words, as described above, the slits may define flaps, which are arranged in a circumferential manner adjacent to each other and merely being separated by the slits. These flaps, which may have an essentially triangular shape, are only attached to the end surface at one end of the flap and a corresponding free end is arranged at an intersection of the slits. It is at such intersection where the flap ends may be truncated or rounded, so as to provide an (a small) opening towards the inner cavity. This further facilitates the advancing of the catheter and further reduces friction at the free ends. Accordingly, a more reliable biasing towards the open position as well as a more reliable closing of the end cap towards the inner cavity may be achieved.

### Brief description of the drawings

The present disclosure will be more readily appreciated by reference to the following detailed description when being considered in connection with the accompanying drawings in which:
Figure 1 shows a schematic depiction of a stent according to the invention in a longitudinal section;
Figure 2 shows a schematic reduced depiction of the stent according to Figure 1 from the distal end region to the proximal end region;
Figure 3 shows a blow-up of the wall of the stent body with a first layer configuration;
Figure 4 shows a blow-up of the wall of the stent body with an alternative first layer configuration;
Figure 5 shows a blow-up of the wall of the stent body with a first layer configuration according to Figure 1;
Figure 6 shows a schematic end section of a catheter having an end cap at a distal end in a longitudinal section; and
Figure 7 shows the embodiment according to Figure 6 seen from the distal end and in a longitudinal direction.

### Detailed description of preferred embodiments

In the following, the invention will be explained in more detail with reference to the accompanying figures. In the Figures, like elements are denoted by identical reference numerals and repeated description thereof may be omitted in order to avoid redundancies.

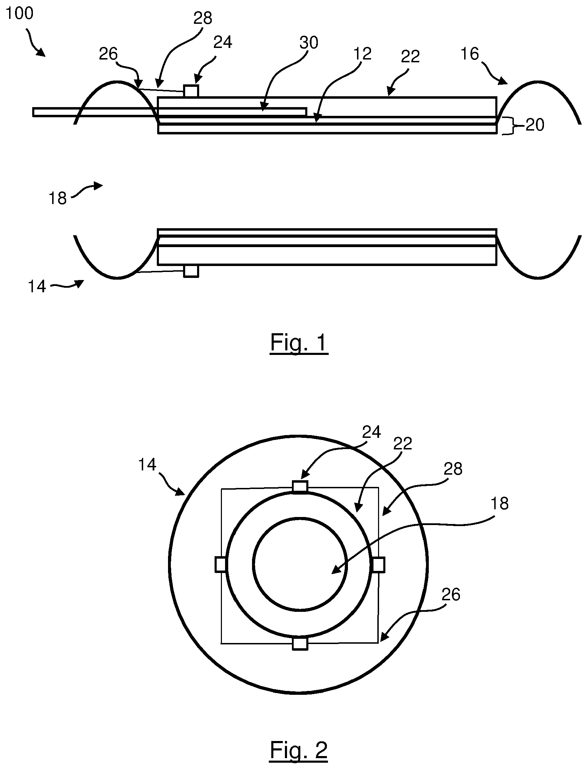

In Figure 1 a schematic depiction of a stent 10 according to the invention is shown in a longitudinal section. The stent 10 is completely expandable and is adapted to be brought to an application site in a hollow organ, in particular the gastrointestinal tract, preferably the esophagus, intestine, primarily rectum, sigma, colon descendens or colon transversum, by conventional application measures. The stent 10 comprises a body 12, which according to the present, non-limiting embodiment is formed of a shape memory allow, preferably nitinol, and comprises an essentially continuous wired mesh-shape (not shown in Figure 1 for improved intelligibility of the various features of the stent 10). The body 12 is hence formed of a resilient, compressible, and collapsible material and may be self-expandable, although other configurations enabling a mechanical expansion may also be provided.

The body 12 has an essentially tubular shape and comprises an essentially continuous cross-sectional area for the largest part of the body 12 and extends in a longitudinal direction from a proximal end 14 towards a distal end 16. The terms proximal and distal are to be understood so as to be closest to an insertion site and closest to an application site, respectively during a surgical procedure for the delivery of the stent 10 towards the application site. The continuous cross-sectional area is delimited by the proximal end region 14 and the distal end region 16, which comprise a varying diameter and radial extension and for the largest part have a radial extension exceeding the radial extension of the continuous cross-sectional area of the body 12. The larger radial extension facilitates securing the stent 10 during deployment in a hollow organ and may furthermore provide that e.g. a lesion or suture is arranged between said proximal end 14 and distal end 16. Thereby, the lesion or suture may be effectively isolated. Both the proximal end 14 and the distal end 16 have a toroidal or mushroom shape, which has a rounded or convex surface and provides an extent of adaptability to the surrounding tissue at the application site with sufficient radial extension to ensure that the stent 10 is properly secured in place.

From the proximal end 14 to the distal end 16 the body 12 or wall thereof furthermore defines a continuous fluid passageway 18. Said passageway 18 is sealed-off towards the inner wall of the hollow organ at the application site by means of a first layer 20, which is liquid-tight and may e.g. be formed of a silicon-based material. The first layer 20 ensures that an isolated lesion or leaky suture is no longer susceptible to be contaminated with the contents of the hollow organ, e.g. the intestine, and vice versa, such that potential high-risk medical complications may be effectively avoided. The sealing towards the inner wall of the hollow organ is furthermore facilitated by a second layer 22, which surrounds the first layer 20 and may be formed of a biocompatible foam or sponge material so as to provide sufficient shapeability and conformability to the local tissue and anatomic structure at the application site. Thereby, such material may also assist in mechanically securing the stent 10 at the desired application site. The continuous cross-sectional area preferably defines a first predefined region of the first layer 20 and a second predefined region of the second layer 22.

By means of the passageway 18 and the improved sealing provided by the end regions 14, 16 as well as the first layer 20 and the second layer 22, normal functioning of the hollow organ may be established while healing of lesions or leaky sutures, for example, anastomosis, is facilitated.

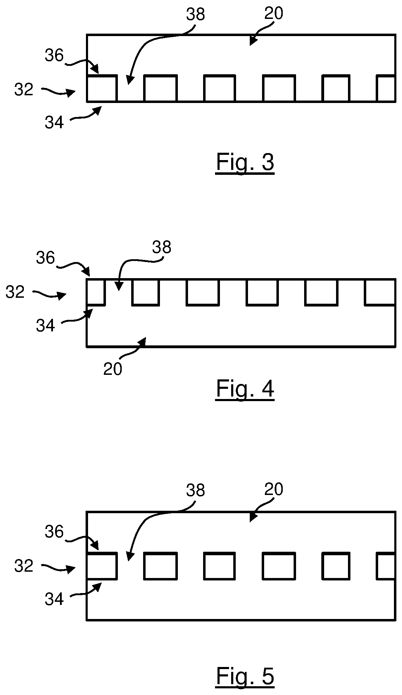

In the present embodiment, the body 12 or the region of the body 12 having a continuous cross-sectional area is fully embedded in the first layer 20. The mesh-shape structure of the body 12 allows that the material of the first layer may extend radially from one side of the wall of the body 12 to an opposing side of said wall, e.g. from an inner wall to an outer wall surface, as will be described herein below with regard to Figures 3 to 5 in further detail. By means of the embedded configuration the first layer 20 is mechanically secured to the body 12 and structural stability of the first layer 20 may be improved. Therefore, proper functioning of the stent 10 and the liquid-tight isolation of the lesion or suture may be ensured even after prolonged application time or deployment at complex tissue structures. Furthermore, the embedding establishes that tissue in-growth towards or around the mesh-shaped body 12 may be effectively avoided or at least reduced.

Proper positioning and functioning of the second layer 22 is furthermore ensured by means of a plurality of eyelets 24 formed by a corresponding first thread portion and arranged equally spaced-apart along an outer circumference of the second layer 22. The eyelets 24 may be formed e.g. by a suturing material, such as PP, PE or PTFE and may be formed as a braided material. Each eyelet 24 is furthermore connected to the body 12 via respective connection points 26 using a second thread portion 28, wherein the first thread portion 24 and the second thread portion 28 are preferably formed of a single continuous thread. The connection points 26 are arranged at the proximal end region 14, preferably at respective points having a larger radial extension than the radial extension at the eyelet 24.

The mechanical fixation provides that the second layer 22 may be safely removed together with the other components of the stent 10, when the application is terminated, avoiding that the second layer 22 may remain in the hollow organ due to potential tissue in-growth. Furthermore, the mechanical fixation facilitates proper positioning of the second layer 22 during deployment of the stent 10.

Also shown in Figure 1 is a cannula 30, which is received at the proximal end region 14 and is introduced via a wall of the body 12 so as to be accommodated along the body 12 between the proximal end region 14 and the distal end region 16. At the proximal end 14, no first layer 20 or second layer 22 is provided for easy of introduction of the cannula 30 and manufacturing of the overall stent 10. However, a liquid-tight foil (not shown) may be connected to the first layer 20 at this region and may ensure proper sealing also in this region e.g. by having a tubular or conical shape, thereby covering the entire (inner) circumference of the toroidally-shaped proximal end region 14.

In Figure 2 it is shown that the second thread portion 28 may be alternated between the eyelets 24 and the connection points 26, forming a zig-zag pattern. In the Figure, the different levels have been reduced for simplification (and the first layer 20 and the stent body 10 are not explicitly shown for improved overview), but it is to be understood that the connection points 26 are arranged at curvature of the proximal end region 14, having a gradually increasing radial extension. Furthermore, the body 12 is preferably formed as a mesh-shaped body 12, such that the connection points 26 may be formed by intersecting wires or struts and the second thread portion 28 may either be knotted or tied to said intersections or be guided or laced around said intersections, forming a loop between two adjacent eyelets 24.

In the present example, four eyelets 24 are provided, the eyelets 24 being equally spaced apart along the circumference of the second layer 22. The eyelets 24 may be formed as protruding loops of the corresponding first thread portion 24, which is arranged within the second layer 22 between said eyelets 24. The connection points 26 are also equally spaced-apart along the corresponding circumference of the body 12 at the proximal end region 14, each connection point 26 being in the middle between two adjacent, neighboring eyelets 24 yet with a longitudinal offset. Although the present embodiment provides an advantageous even distribution of forces and even mechanical fixation without providing constrictions of the second layer 22 or body 12, it will be understood that other connection patterns may be provided and eyelet 24 and/or connection point 26 configurations may be provided, depending on the requirements of the stent 10 and the application site.

In Figures 3 to 5 alternative configurations of the first layer 20 are shown with respect to the wall 32 of the body 12. Accordingly, said wall comprises an inner wall surface 34 and an outer wall surface 36 in reference to the passageway 18 and comprises a plurality of (continuous) openings 38, which may e.g. be formed by a mesh-shaped configuration of the body 12.

In all of the embodiments, the body 12 or wall 32 thereof is embedded by the first layer 20. According to the embodiment of Figure 3, the material of the first layer 20 is primarily arranged at an outer wall surface 36, yet protrudes into the openings 38, such that the first layer 20 is mechanically secured to the wall 32, but the passageway 18 may essentially be free of any first layer 20. Thereby, the diameter of the passageway 18 is not affected by the first layer 20 and obstruction of the passageway 18 is essentially avoided, even in the case of a (partial) loosening of the first layer 20.

An alternative configuration is depicted in Figure 4, wherein the material of the first layer 20 is primarily arranged at an inner wall surface 34. This enables that the radial extension of the second layer 22 may e.g. be increased or may be advantageous, when a liquid-tight foil is implemented at the proximal end region 14.

Figure 5 shows a configuration, wherein the entire wall 32, i.e. the outer wall surface 36 and inner wall surface 34 are embedded by the material of the first layer 20, which is also schematically depicted in the embodiment according to Figure 1.

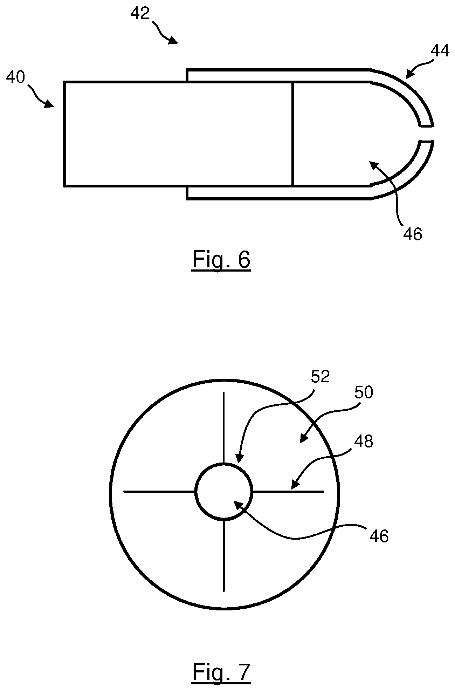

In Figure 6 an end section of a catheter 40 having an end cap 42 at a distal end is schematically shown in a longitudinal section. The catheter 40 and end cap 42 may be part of a delivery system for delivering and deploying a stent 10 to a target anatomic region, such stent 10 (not shown) being retained in the catheter 40 in a compressed and preferably collapsed state during delivery and navigation towards the application site.

The end cap 42 is formed of a flexible and preferably resilient or elastic material and defines an inner cavity 46 for receiving part of the catheter 40 during deployment of the stent 10. The inner cavity 46 hence ensures that the catheter 40 may be advanced through the end cap 42, when the catheter 40 is at the appropriate position. The advancing of the catheter 40 out of the end cap 42 is furthermore facilitated by an outwardly convex surface 44 at the distal end of the end cap 42, which comprises a plurality of slits 48, preferably four slits 48, as shown in further detail in Figure 7.

Accordingly, the four slits 48 of the end cap 42 may be equally circumferentially spaced-apart from each other, forming essentially a cross shape, wherein the slits intersect or interface each other in a radial center point of the end cap 42. The slits 48 form four equally shaped flaps 50 being of a triangular shape, which are only connected at one side of said shape and having a free end at the intersecting point of the flaps 50. At the intersecting point, the flaps 50 comprise a truncated region, which may be rounded so as to provide an (a small) opening towards the inner cavity 46. Thereby, proper biasing during closing and opening of the end cap 42 may be facilitated and initial advancement of the catheter 40 through and out of the end cap 42 may be supported by the small opening, reducing initial resistance of the flaps 50 due to resilience of the material.

The convex shape and the provision of a plurality of slits facilitates navigation and deployment of the stent 10 and furthermore allows using a smaller bead at a distal end of a guide wire. Furthermore, the end cap 42 may be dimensioned smaller compared with conventional solutions, such that the surrounding tissue at the application site is not adversely affected by the end cap 42 and the dimensioning may be fully adapted to the dimensioning of the stent 10.

It will be obvious for a person skilled in the art that these embodiments and items only depict examples of a plurality of possibilities.

### List of reference numerals

- 10: Stent
- 12: Body
- 14: Proximal end region
- 16: Distal end region
- 18: Passageway
- 20: First layer
- 22: Second layer
- 24: Eyelet or first thread portion
- 26: Connection point
- 28: Second thread portion
- 30: Cannula
- 32: Wall
- 34: Inner wall surface
- 36: Outer wall surface
- 38: Opening
- 40: Catheter
- 42: End cap
- 44: Convex surface
- 46: Inner cavity
- 48: Slit
- 50: Flap
- 52: Truncated region

## Claims

1. A stent (10) for introduction into a hollow organ of the human or animal body, preferably into the gastrointestinal tract, in particular the intestine, comprising
- a radially expandable body (12) having a wall (32) defining an inner fluid passageway (18) from one end of the body (12) to a longitudinally opposing end of the body (12),
- a liquid-tight and flexible first layer (20), the first layer (20) covering a surface (34, 36) of said wall (32) along its entire circumference and along a first predefined region in a longitudinal direction of said body (12), and
- a resilient porous second layer (22), the second layer (22) covering an outer surface (36) of said wall (32) along its entire circumference and along a second predefined region in a longitudinal direction of said body (12) and at least partially covering the first layer (20),
wherein the second layer (22) is mechanically secured to the body (12),
**characterized in that**
the second layer (22) is mechanically attached to the body (12) by means of at least one thread, a first thread portion (24) being arranged in the second layer (22) so as to provide at least one eyelet (24) at an outer surface of said second layer (22) and a second thread portion (28) connecting the at least one eyelet (24) to a respective connection point (26) at an adjacent end region (14, 16) of the body (12) not being covered by the second layer (22).

2. A stent (10) according to claim 1, wherein the first layer (20) is arranged at said wall surface (34, 36) and extends at least partially through said wall (32) in a radial direction.

3. The stent (10) according to claim 1 or 2, wherein the first layer (20) covers an inner surface (34) and an outer surface (36) of said wall (32).

4. The stent (10) according to any of the preceeding claims, wherein the first predefined region and/or the second predefined region corresponds to a region of the body (12) having an essentially continuous cross-sectional area.

5. The stent (10) according to any of the preceding claims, wherein the first predefined region and/or the second predefined region is longitudinally delimited by at least one end region (14, 16) of the body, the at least one end region (14, 16) having an enlarged radial extension.

6. The stent (10) according to claim 5, wherein, in a longitudinal section of the body (12) the at least one end region has a mushroom shape, dome shape, toroidal shape, or donut shape, and/or the body (12) has a barbell shape.

7. The stent (10) according to claim 5 or 6, wherein both the first predefined region and the second predefined region are delimited by both of the opposing end regions (14, 16),
wherein the second layer (22) is preferably mechanically secured in a form fitting manner by the opposing end regions (14, 16) in the longitudinal direction.

8. The stent (10) according to any of the preceding claims, wherein at least two eyelets (24) are provided by the first thread portion (24), said eyelets (24) being circumferentially spaced apart,
wherein said eyelets (24) are preferably equally spaced apart along the circumference and/or wherein between three and six or four eyelets (24) are provided by the first thread portion (24).

9. The stent (10) according to claim 8, wherein the second thread portion (28) alternates between connection points (26) and eyelets (24) being adjacent to each other and/or wherein each connection point (26) is connected to two adjacent neighboring eyelets (24) by means of the second thread portion (28).

10. The stent (10) according to claim 8 or 9, wherein each connection point (26) is arranged essentially equidistantly to two adjacent neighboring eyelets (24).

11. The stent (10) according to any of the preceding claims, wherein the first thread portion (24) is arranged within the material of the second layer (22) between adjacent eyelets (24).

12. The stent (10) according to any of the preceding claims, wherein the first thread portion (24) and the second thread portion (28) are formed of a single thread.

13. The stent (10) according to any of the preceding claims, wherein the adjacent end region (14, 16) is configured for accommodating a cannula (30).

14. The stent (10) according to any of the preceding claims, further comprising a cannula (30) being arranged between the second layer (22) and the first layer (20) and essentially being accommodated outside of the body (12).

15. A delivery system for delivering and deploying a stent (10) to a target anatomic region, comprising a catheter (40) having a stent (10) according to any of the preceding claims in a compressed state.

16. The delivery system according to claim 15, wherein the catheter (40) comprises a distal end cap (42) made of a flexible material, wherein the end cap (42) defines an inner cavity (46) and has a convex rounded end surface (44) in a longitudinal direction, the end surface having at least three, preferably four slits (48) from its outer surface towards the inner cavity (46) that are equally spaced apart in the circumferential direction, wherein a shape of the end surface defined by two circumferentially adjacent slits (48) is preferably truncated at its free end.

## Patentansprüche

1. Stent (10) zum Einführen in ein Hohlorgan des menschlichen oder tierischen Körpers, vorzugsweise in den Magen-Darm-Trakt, insbesondere den Darm, umfassend
- einen radial expandierbaren Körper (12) mit einer Wand (32), die einen inneren Fluidkanal (18) von einem Ende des Körpers (12) zu einem längs gegenüberliegenden Ende des Körpers (12) definiert,
- eine flüssigkeitsdichte und flexible erste Schicht (20), wobei die erste Schicht (20) eine Fläche (34, 36) der Wand (32) entlang ihres gesamten Umfangs und entlang eines ersten vordefinierten Bereichs in einer Längsrichtung des Körpers (12) bedeckt, und
- eine elastische poröse zweite Schicht (22), wobei die zweite Schicht (22) eine Außenfläche (36) der Wand (32) entlang ihres gesamten Umfangs und entlang eines zweiten vordefinierten Bereichs in einer Längsrichtung des Körpers (12) bedeckt und zumindest teilweise die erste Schicht (20) bedeckt,
wobei die zweite Schicht (22) an dem Körper (12) mechanisch befestigt ist,
**dadurch gekennzeichnet, dass**
die zweite Schicht (22) mittels mindestens eines Fadens mechanisch an dem Körper (12) angebracht ist, wobei ein erster Fadenabschnitt (24) in der zweiten Schicht (22) angeordnet ist, um mindestens eine Öse (24) an einer Außenfläche der zweiten Schicht (22) bereitzustellen, und wobei ein zweiter Fadenabschnitt (28) die mindestens eine Öse (24) mit einem entsprechenden Verbindungspunkt (26) an einem benachbarten Endbereich (14, 16) des Körpers (12) verbindet, der nicht von der zweiten Schicht (22) bedeckt ist.

2. Stent (10) nach Anspruch 1, wobei die erste Schicht (20) an der Wandfläche (34, 36) angeordnet ist und sich zumindest teilweise in einer radialen Richtung durch die Wand (32) erstreckt.

3. Stent (10) nach Anspruch 1 oder 2, wobei die erste Schicht (20) eine Innenfläche (34) und eine Außenfläche (36) der Wand (32) bedeckt.

4. Stent (10) nach einem der vorhergehenden Ansprüche, wobei der erste vordefinierte Bereich und/oder der zweite vordefinierte Bereich einem Bereich des Körpers (12) entspricht, der eine im Wesentlichen kontinuierliche Querschnittsfläche aufweist.

5. Stent (10) nach einem der vorhergehenden Ansprüche, wobei der erste vordefinierte Bereich und/oder der zweite vordefinierte Bereich längs durch mindestens einen Endbereich (14, 16) des Körpers begrenzt ist, wobei der mindestens eine Endbereich (14, 16) eine vergrößerte radiale Ausdehnung aufweist.

6. Stent (10) nach Anspruch 5, wobei in einem Längsschnitt des Körpers (12) der mindestens eine Endbereich eine Pilzform, eine Kuppelform, eine Toroidform oder eine Donutform aufweist und/oder der Körper (12) eine Hantelform aufweist.

7. Stent (10) nach Anspruch 5 oder 6, wobei sowohl der erste vordefinierte Bereich als auch der zweite vordefinierte Bereich durch die beiden gegenüberliegenden Endbereiche (14, 16) begrenzt sind,
wobei die zweite Schicht (22) vorzugsweise durch die gegenüberliegenden Endbereiche (14, 16) in der Längsrichtung formschlüssig mechanisch befestigt ist.

8. Stent (10) nach einem der vorhergehenden Ansprüche, wobei mindestens zwei Ösen (24) durch den ersten Fadenabschnitt (24) bereitgestellt sind, wobei die Ösen (24) umlaufend voneinander beabstandet sind,
wobei die Ösen (24) vorzugsweise gleichmäßig entlang des Umfangs beabstandet sind und/oder wobei zwischen drei und sechs oder vier Ösen (24) durch den ersten Fadenabschnitt (24) bereitgestellt sind.

9. Stent (10) nach Anspruch 8, wobei der zweite Fadenabschnitt (28) zwischen Verbindungspunkten (26) und Ösen (24), die benachbart zueinander sind, wechselt und/oder wobei jeder Verbindungspunkt (26) mittels des zweiten Fadenabschnitts (28) mit zwei benachbarten Ösen (24) verbunden ist.

10. Stent (10) nach Anspruch 8 oder 9, wobei jeder Verbindungspunkt (26) im Wesentlichen äquidistant zu zwei benachbarten Ösen (24) angeordnet ist.

11. Stent (10) nach einem der vorhergehenden Ansprüche, wobei der erste Fadenabschnitt (24) innerhalb des Materials der zweiten Schicht (22) zwischen benachbarten Ösen (24) angeordnet ist.

12. Stent (10) nach einem der vorhergehenden Ansprüche, wobei der erste Fadenabschnitt (24) und der zweite Fadenabschnitt (28) aus einem einzigen Faden gebildet sind.

13. Stent (10) nach einem der vorhergehenden Ansprüche, wobei der benachbarte Endbereich (14, 16) zum Aufnehmen einer Kanüle (30) eingerichtet ist.

14. Stent (10) nach einem der vorhergehenden Ansprüche, ferner umfassend eine Kanüle (30), die zwischen der zweiten Schicht (22) und der ersten Schicht (20) angeordnet ist und im Wesentlichen außerhalb des Körpers (12) aufgenommen ist.

15. Zuführsystem zum Zuführen und Entfalten eines Stents (10) zu einem anatomischen Zielbereich, umfassend einen Katheter (40) mit einem Stent (10) nach einem der vorhergehenden Ansprüche in einem komprimierten Zustand.

16. Zuführsystem nach Anspruch 15, wobei der Katheter (40) eine distale Endkappe (42) aus einem flexiblen Material umfasst, wobei die Endkappe (42) einen inneren Hohlraum (46) definiert und eine konvexe gerundete Endfläche (44) in einer Längsrichtung aufweist, wobei die Endfläche mindestens drei, vorzugsweise vier Schlitze (48) von ihrer Außenfläche in Richtung des inneren Hohlraums (46) aufweist, die in der Umfangsrichtung gleichmäßig beabstandet sind, wobei eine Form der Endfläche, die durch zwei umlaufend benachbarte Schlitze (48) definiert ist, vorzugsweise an ihrem freien Ende abgeschnitten ist.

## Revendications

1. Endoprothèse (10) destinée à être introduite dans un organe creux du corps d'un être humain ou d'un animal, de préférence dans le tractus gastro-intestinal, en particulier l'intestin, comprenant :
- un corps extensible radialement (12) présentant une paroi (32) définissant un passage de fluide intérieur (18) depuis une extrémité du corps (12) vers une extrémité opposée longitudinalement du corps (12),
- une première couche étanche au liquide et flexible (20), la première couche (20) recouvrant une surface (34, 36) de ladite paroi (32) le long de toute sa circonférence et le long d'une première région prédéfinie dans une direction longitudinale dudit corps (12), et
- une seconde couche poreuse résiliente (22), la seconde couche (22) couvrant une surface extérieure (36) de ladite paroi (32) le long de toute sa circonférence et le long d'une seconde région prédéfinie dans une direction longitudinale dudit corps (12) et recouvrant au moins partiellement la première couche (20),
dans lequel la seconde couche (22) est mécaniquement fixée au corps (12),
**caractérisée en ce que**
la seconde couche (22) est mécaniquement rapportée sur le corps (12) au moyen d'au moins un fil, une première partie de fil (24) étant agencée dans la seconde couche (22) de façon à fournir au moins un œillet (24) sur une surface extérieure de ladite seconde couche (22) et une seconde partie de fil (28) reliant l'au moins un œillet (24) à un point de liaison respectif (26) au niveau d'une région d'extrémité adjacente (14, 16) du corps (12) qui n'est pas couverte par la seconde couche (22).

2. Endoprothèse (10) selon la revendication 1, dans laquelle la première couche (20) est agencée sur ladite surface de paroi (34, 36) et s'étend au moins partiellement à travers ladite paroi (32) dans une direction radiale.

3. Endoprothèse (10) selon la revendication 1 ou 2, dans laquelle la première couche (20) recouvre une surface intérieure (34) et une surface extérieure (36) de ladite paroi (32).

4. Endoprothèse (10) selon l'une quelconque des revendications précédentes, dans laquelle la première région prédéfinie et/ou la seconde région prédéfinie correspond à une région du corps (12) présentant une superficie transversale essentiellement continue.

5. Endoprothèse (10) selon l'une quelconque des revendications précédentes, dans laquelle la première région prédéfinie et/ou la seconde région prédéfinie est longitudinalement délimitée par au moins une région d'extrémité (14, 16) du corps, l'au moins une région d'extrémité (14, 16) présentant une extension radiale élargie.

6. Endoprothèse (10) selon la revendication 5, dans laquelle, dans une coupe longitudinale du corps (12), l'au moins une région d'extrémité présente une forme de champignon, une forme de dôme, une forme toroïdale, ou une forme de donut, et/ou le corps (12) présente une forme d'haltère.

7. Endoprothèse (10) selon la revendication 5 ou 6, dans laquelle la première région prédéfinie et la seconde région prédéfinie sont délimitées par les deux régions d'extrémité opposées (14, 16),
dans laquelle la seconde couche (22) est de préférence mécaniquement fixée par assemblage par forme par les régions d'extrémité opposées (14, 16) dans la direction longitudinale.

8. Endoprothèse (10) selon l'une quelconque des revendications précédentes, dans laquelle au moins deux œillets (24) sont fournis par la première partie de filage (24), lesdits œillets (24) étant espacés de manière circonférentielle,
dans laquelle lesdits œillets (24) sont de préférence espacés de manière équidistante le long de la circonférence et/ou dans laquelle entre trois et six ou quatre œillets (24) sont fournis par la première partie de fil (24).

9. Endoprothèse (10) selon la revendication 8, dans laquelle la seconde partie de fil (28) alterne entre des points de liaison (26) et des œillets (24) adjacents les uns aux autres et/ou dans laquelle chaque point de liaison (26) est relié à deux œillets adjacents (24) au moyen de la seconde partie de fil (28).

10. Endoprothèse (10) selon la revendication 8 ou 9, dans laquelle chaque point de liaison (26) est agencé de manière essentiellement équidistante de deux œillets adjacents (24).

11. Endoprothèse (10) selon l'une quelconque des revendications précédentes, dans laquelle la première partie de fil (24) est agencée dans le matériau de la seconde couche (22) entre des œillets adjacents (24).

12. Endoprothèse (10) selon l'une quelconque des revendications précédentes, dans laquelle la première partie de fil (24) et la seconde partie de fil (28) sont constituées d'un unique fil.

13. Endoprothèse (10) selon l'une quelconque des revendications précédentes, dans laquelle la région d'extrémité adjacente (14, 16) est configurée pour recevoir une canule (30).

14. Endoprothèse (10) selon l'une quelconque des revendications précédentes, comprenant en outre une canule (30) agencée entre la seconde couche (22) et la première couche (20) et étant essentiellement reçue à l'extérieur du corps (12).

15. Système de distribution pour distribuer et déployer une endoprothèse (10) vers une région anatomique cible, comprenant un cathéter (40) doté d'une endoprothèse (10) selon l'une quelconque des revendications précédentes, dans un état comprimé.

16. Système de distribution selon la revendication 15, dans lequel le cathéter (40) comprend un capuchon d'extrémité distale (42) réalisé en un matériau flexible, dans lequel le capuchon d'extrémité (42) définit une cavité intérieure (46) et présente une surface d'extrémité arrondie convexe (44) dans une direction longitudinale, la surface d'extrémité présentant au moins trois, de préférence quatre fentes (48) à partir de sa surface extérieure vers la cavité intérieure (46) qui sont espacées de manière égale dans la direction circonférentielle, dans lequel une forme de la surface d'extrémité définie par deux fentes (48) adjacentes de manière circonférentielle est de préférence tronquée à son extrémité libre.
